# EUROPEAN PATENT APPLICATION

(11) **EP 4 043 543 A1**
(43) Date of publication of application: **17.08.2022**
(21) Application number: 22156765.4
(22) Date of filing: 15.02.2022
(51) Int. Cl.: C12M 1/00

(54) **ARTIFICIAL LIGHTING SYSTEM FOR THE LIGHTING OF A PHOTOBIOREACTOR**

(30) Priority: 15.02.2021 IT 202100003308
(71) Applicant: BIOSYNTEX S.R.L., 40123 Bologna (IT)
(72) Inventor: CAMPANI, Cristian, 40123 BOLOGNA (IT); LESCI, Giorgio Isidoro, 40123 BOLOGNA (IT)
(74) Representative: Rigamonti, Dorotea

(57) **Abstract**

The subject of the present invention is a kit for the lighting of a photobioreactor (PBR) which comprises:
- at least one Artificial Lighting System (ALS) (1);
- at least one light diffusing element;
where said at least one ALS (1) comprises an external face (3) and an internal face (4), where on said external face (3) a plurality of blades (2) are placed and on said internal face (4) a plurality of LED modules (5 are placed), wherein said plurality comprises at least one LED in each of the RED, WHITE and BLUE channels; where said at least one ALS (1) is positioned at the top and / or at the bottom of at least one of said light diffusing elements and said external face (3) faces outwards, said internal face (4) faces towards the interior of said light diffusing element and said ALS acts as a sealing element of said light diffusing element.

Said diffuser element is made of a material transparent to light and is at least partially coated with a reflector which is a metal film with low thickness and high surface reflectance in which slits are carved.

A further object of the present invention is a method for controlling the lighting system according to the present invention.

## Description

Algae crops need to receive light radiation to metabolize CO₂, releasing O₂ with the photosynthesis process. Algal proliferation is favoured by particular feeding and lighting regimes, proliferation that allows the exploitation for the extraction of chemical substances to be used, for example, for the synthesis of fuels and fertilizers.

The algal culture can be exposed directly to solar radiation or indirectly. In the latter case, the light radiation is concentrated through optical devices, mirrors, or lenses, and possibly conveyed through optical fibres or light tubes to the culture medium.

WO2020031128 describes a system based exclusively on natural light where the illumination of the crop is optimized by optical filters that transmit only the most active spectral portion for photosynthetic purposes, reflecting and / or absorbing the spectral portion scarcely useful for photosynthetic purposes.

To maximize algal growth, avoiding natural light-dark cycles dependency but at the same time maintaining process efficiency, it is useful to integrate natural lighting with artificial lighting.

Therefore, the object of the present invention is a bioreactor lighting system for algal culture and a method for controlling it with surprisingly advantageous characteristics.

### Description of the invention

A first object of the present invention is a kit for the lighting of a photobioreactor (PBR) which comprises:
- at least one Artificial Lighting System (ALS);
- at least one light diffusing element;
where said at least one ALS comprises an external face and an internal face, where a multiplicity of blades are placed on said external face and a multiplicity of LED modules are placed on said internal face; where said at least one ALS is positioned at the top and / or at the bottom of at least one of said light diffuser and said external face faces outwards, said internal face faces inwards of said light diffuser element and said ALS acts as a sealing element of said light diffusing element.

In one embodiment, said plurality of LEDs comprises at least one LED in each of the RED, WHITE and BLUE channels.

In one embodiment, said light diffusing element is a light diffusing tube.

In one embodiment, said light diffusing element is a light diffusing panel.

Inside said light diffusing element there is a reflector which is a metal film on which there are slits arranged according to a geometry that has proved to be particularly advantageous.

A further object of the present invention is a method of controlling the lighting system, where said method is an automated method for adjusting the intensity and spectrum of light of the LED modules included in said ALS.

### Drawings

**Figure 1****:** an embodiment of an ALS according to the present invention. (A) perspective view from above; (B) perspective view from below; (C) lateral view.
**Figure 2****:** an embodiment of the diffuser element which is a light diffusing tube. (A) side schematic view; (B) simulation of light radiation diffusion; (C) emission spectrum of light radiation.
**Figure 3****:** an embodiment of the diffuser element which is a light diffusing tube. (A) side schematic view; (B) simulation of light radiation diffusion; (C) emission spectrum of light radiation.
**Figure 4****:** light spectrum obtained in a PBR with an embodiment of the kit according to the present invention.

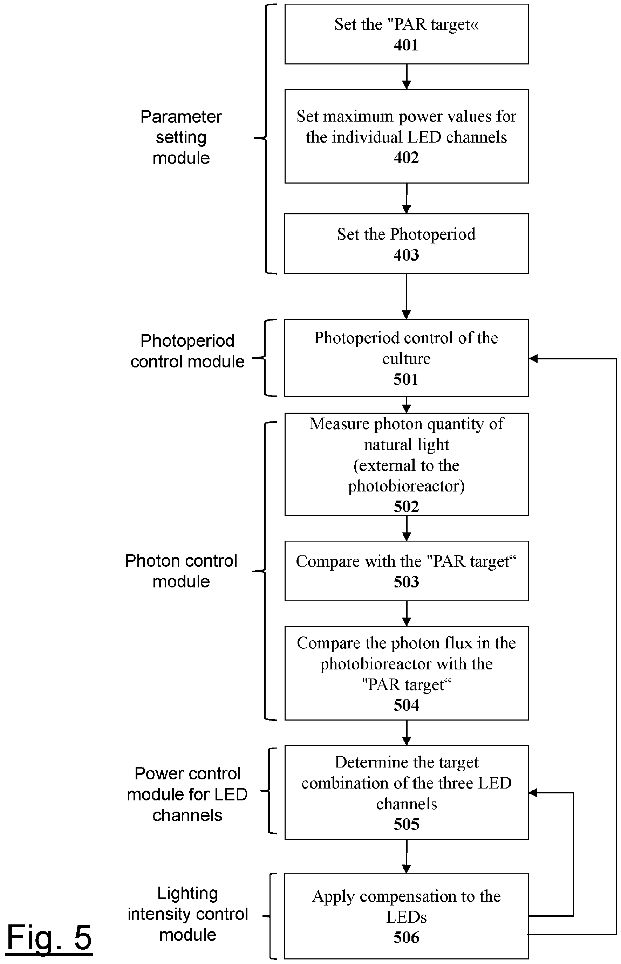

**Figure 5****:** flow diagram of the ALS control method according to the present invention.
**Figure 6****:** an embodiment of the diffuser element which is a light diffusing panel. (A) side schematic view; (B) perspective view of three panels inserted in a tank; (C) perspective view in section of said three panels inserted in the tank.

### Detailed description of the invention

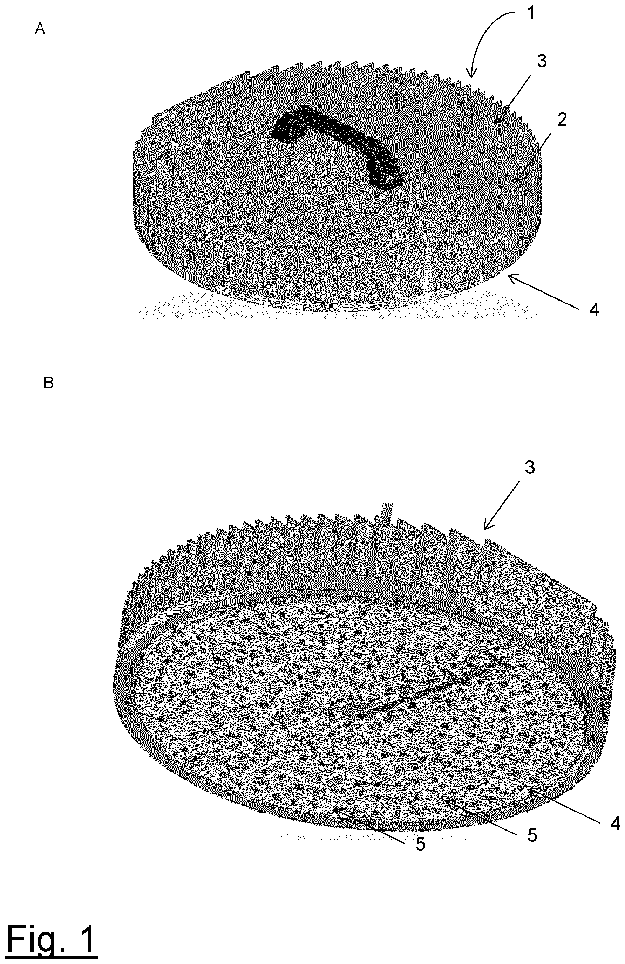

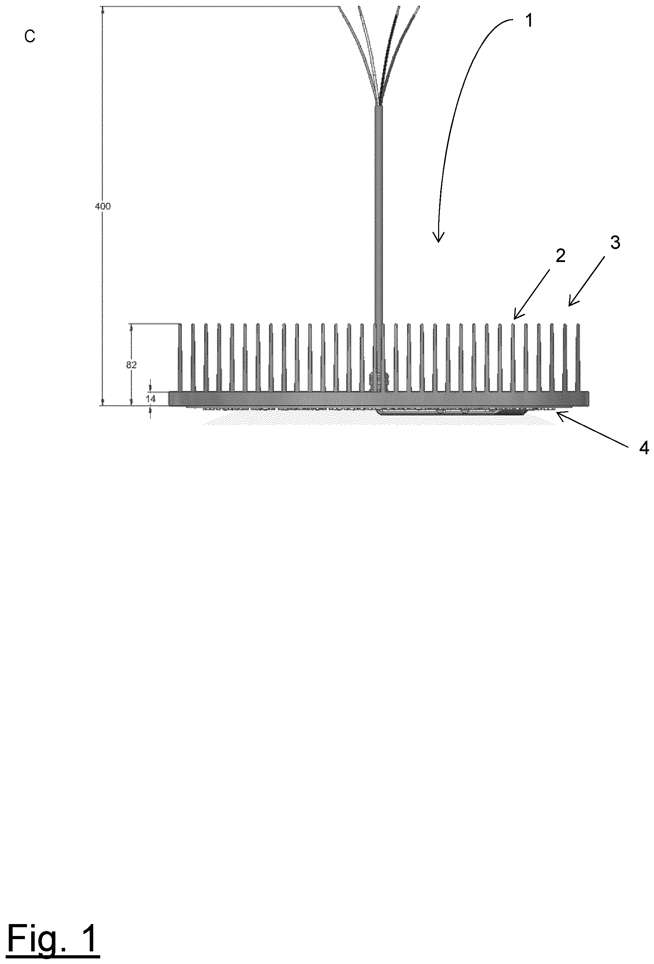

The kit for lighting a photobioreactor (PBR) for algal culture according to the present invention comprises, with reference to Figure 1, at least one Artificial Lighting System (ALS) 1 which, in addition to housing LED modules, performs a dual function: it is a sealing element of the light diffusing element and acts as a dissipating element of the heat generated by the LED modules housed on it.

Said ALS 1 comprises, Figure 1A, 1B, an external face 3 and an internal face 4. Said ALS 1 is positioned on the top and / or at the bottom of a light diffusing element, sealing it.

When said ALS 1 is positioned on the top and / or at the bottom of a light diffusing element, said external face 3 faces outward, said internal face 4 faces inward of said light diffuser element.

Said external face 3 acts as a dissipating element and therefore has a geometry conveniently studied for the purpose. In the embodiment of figure 1, said external face 3 comprises a plurality of blades 2. Figure 1C shows a side view of said ALS, where said blades 2 are highlighted.

In one embodiment, the surface of said blades is smooth.

In an alternative embodiment, the surface of said blades is corrugated, thus causing an increase in the exposed surface of the radiant material which results in a greater heat exchange area with the same dimensions of the blade itself.

Conveniently, said ALS is made of a metallic material, preferably it is aluminium.

The LED modules 5 are housed on said internal face 4.

These LED modules 5 are a combination of LEDs with specific wavelengths, dividing the LED modules into three different channels, RED, WHITE, BLUE channel.

In a preferred embodiment, said LED modules 5 comprise the following LEDs:
- RED channel, for example Hyper Red (635-666 nm) type OSLON SQUARE 120°;
- WHITE channel, for example White 3000K type OSLON SQUARE;
- BLUE channel, for example Deep Blue type LiteON 120° (425-455nm) and Blue type CREE XPE-2 120° (450-480nm) installed in series, preferably in a 1: 1 ratio.

The authors of the present invention have developed an algorithm which, taking into consideration the thermal and energy balance of each single LED emitter, established a radiant mass with the geometry described here. In particular, the following parameters were considered:
- electrical characteristic of the individual LED illuminators, differential forward voltage, specific to each LED based on the electro-chemical properties. In one embodiment, said parameters are defined as follows:
   o forward voltage Led Hyper Red 2,1Vf
   o forward voltage Led White 3000K 2,8Vf
   o forward voltage Led DeepBlue 3.4Vf
   o forward voltage Led Blue 3.1Vf
- electrical intensity values for switching on the LED series per channel, choice determined by the specific emission curve, point of best light intensity / component life ratio and optimal point for specific wavelength emission. In one embodiment, said parameters are defined as follows:
   ∘ RED channel driving current 700mA
   ∘ driving current of WHITE channel 700mA
   ∘ BLUE channel driving current 700mA
- calculation of the electrical powers involved for each channel of the ALS illuminator. In one embodiment, said parameters are defined as follows:
- RED channel electrical power:
   ∘ n.90 Hyper Red LEDs (635-666 nm) type OSLON SQUARE 120°
   ∘ single LED power calculation: 2.1Vf × 0.7A = 1.47W
   ∘ total power per channel: 90 × 1.47 = 132.3W
- WHITE channel electrical power:
   ∘ N.80 LED White 3000K type OSLON SQUARE 120°
   ∘ Single LED power calculation: 2.8Vf × 0.7A = 1.96W
   ∘ Total power per channel: 80 × 1.96 = 156.8W
- BLUE channel electrical power:
   ∘ N.40 Deep Blue LEDs type LiteON 120° (425-455nm)
   ∘ Deep Blue LED single power calculation: 3.4Vf x 0.7A = 2.38W
   ∘ N.40 Blue type CREE XPE-2 120° (450-480nm)
   ∘ Blue LED single power calculation: 3.1Vf × 0.7A = 2.17W
   ∘ Total power per channel: (40 × 2.38) + (40 × 2.17) = 182W
- Total electrical power: 471W
- LED system conversion efficiency: 60.3% (percentage of power converted to light)
- Total thermal power: 187W (heat to dissipate).

In the embodiment with corrugated blades, an increase in heat exchange is obtained with respect to the embodiment with smooth blades estimated at 30% which allows to dissipate the heat generated by the LED system with less mass.

The kit according to the present invention also comprises at least one light diffusing element which, sealed at the top and / or at the bottom by said ALS, is immersed in a PBR.

Said light diffusing element is made of a material transparent to light, preferably it is PMMA.

In one embodiment, said diffuser element is at least partially coated with a reflector.

In one embodiment, said reflector is a metallic film, preferably a prismatic lattice film which exploits the phenomenon of total internal reflection (OLF). By way of example, said diffusing element is a light diffusing tube of the type described in WO2018167721.

In one embodiment, said diffuser element is at least partly coated with a metal film of low thickness and high surface reflectance in which slits are carved through which the light passes from the light diffuser element to the PBR in which it is immersed.

Therefore, a further object of the present invention is a light diffusing element made of transparent material, preferably of PMMA, internally coated with a metal film of low thickness and high surface reflectance in which slits are carved.

In one embodiment, said diffusing element is a light diffusing tube.

In one embodiment, said diffusing element is a light diffusing panel.

In a preferred form, said diffuser element is a light diffusing tube and said metal film is cut to obtain the desired slots, rolled up to form a cylinder and retained inside the light diffusing tube so that the inside of the light diffusing tube is defined by the metal film and the outside of the light diffusing tube is defined by the transparent material that constitutes the tube itself.

The characteristics of said light diffusing element allow an almost constant distribution of the photon flux density over the entire length of the PBR, allowing to optimize the illumination of the algal suspension in all depth.

In one embodiment, said light diffusing tube has a diameter longer than 300 mm, preferably between 350 and 800 mm, preferably about 400 mm and a length between 1.5 and 5 meters, preferably about 2 meters, or about 3 meters.

In a preferred embodiment, the slots on said light diffusing tube are arranged with a specific geometry, based on the division of the tube into sections along the length of the tube itself. The authors of the present invention have defined a preferred size and distribution geometry of the same slots in the different sections that make up said light diffusing tube.

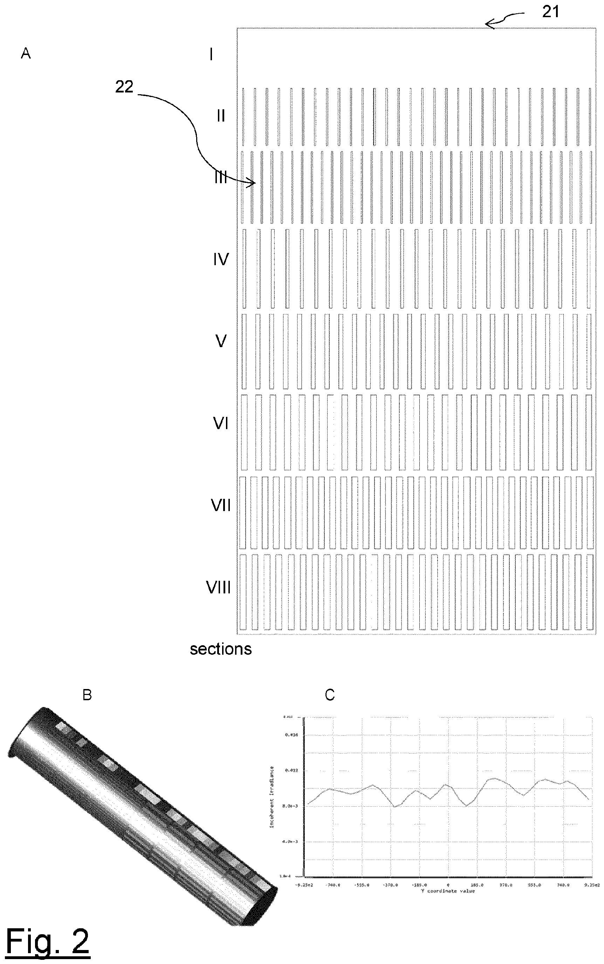

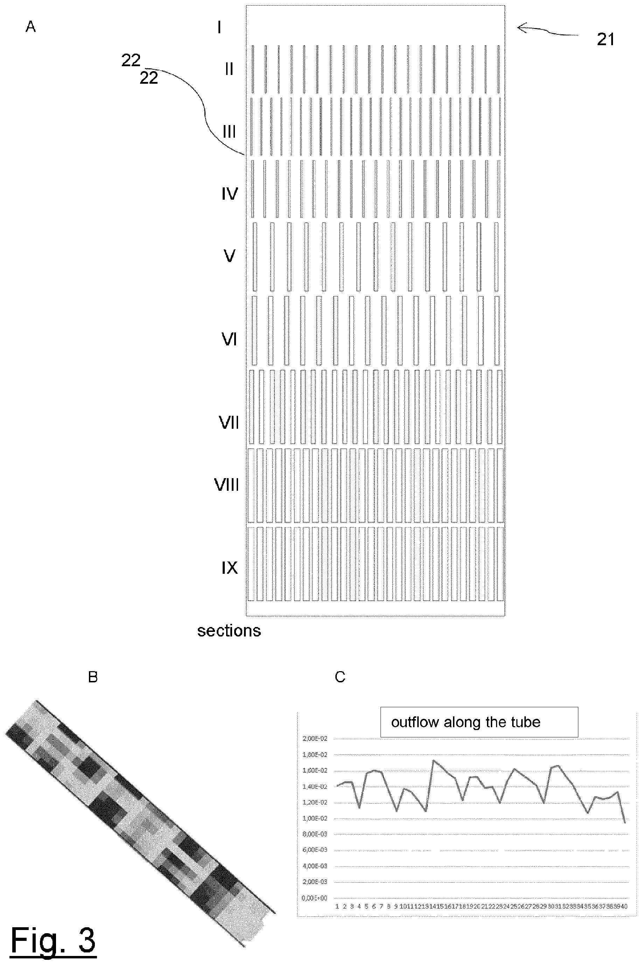

Typically, said light diffusing tube is divided into a number of sections greater than 7. In one embodiment, with reference to Figure 2, said light diffusing tube is 2 m long and is divided into 8 sections. In a further embodiment, with reference to Figure 3, said light diffusing tube is 3 m long and is divided into 9 sections.

The number and geometry of the slits opening on each section is optimized to maximize the uniformity of light distribution along the diffuser tube, where a certain number of slots in one section also indirectly affects the light delivered by the subsequent sections, the less light arrives on subsequent sections, less light can be extracted.

In one embodiment, said slots are rectangular in shape. In one embodiment, the longer side of said rectangle which constitutes said slot has a maximum length equal to the length of said section.

In one embodiment, the dimensions of the smaller side of said rectangle constituting said slot increase as it passes from the top sections of the light diffusing tube to the bottom sections. Preferably, the length of said shorter side is between 3 and 30 mm, even more preferably between 5 and 20 mm.

In one embodiment, the number of slits varies from the top sections of the light diffusing tube to the bottom sections. For example, this number is higher in the top and bottom sections, while it is lower in the middle sections. Said number of slits present on each section is preferably between 0 and 50, or between 15 and 50, or between 20 and 40, or between 25 and 36. In a preferred embodiment, the first top section has no slits. In a preferred form, said number is about 30 in the second top section, about 25 in the central sections, about 30 in the bottom section.

Figure 2A schematically shows a particularly advantageous geometry of slots. In the embodiment of Figure 2A, the light diffusing tube 21 is 2 m long, has a diameter of 400 mm and is divided into 8 sections, I-VIII. Going from the top end to the bottom end of said light diffusing tube, there is a first section without slits 22, a second section with 30 slits 22, a third with 36, a fourth, fifth and a sixth section with about 25 slits 22, a seventh with 32, an octave with 30. Even more preferably, after said first section without slits 22, there is the second section with 30 slits 22, a third with 36, a fourth with 25 a fifth with 26, a sixth with 25, a seventh with 32, an octave with 30.

Again, with reference to figure 2A, the length of the shorter side of said slits is about 5 mm in the second and third sections, to pass to about 15 mm in the fourth and fifth sections, up to about 20 mm in the sixth, seventh, eighth section. In a preferred form, the length of the shorter side of said slits is about 5 mm in the second and third sections, about 10 mm in the fourth, about 14 mm in the fifth, about 20 mm in the remaining three sections. With said distribution of the slits, a light distribution of the type represented in figure 2B, 2C is obtained, where figure 2C represents a perspective view of a light diffusing tube which has a diameter of 400 mm, a length of 2 m, a development of 1212 mm. On the light diffusing tube 21 there are slits 22 and the emission spectrum 23 is indicated. The incoherent irradiation along the entire length of the tube remains almost constant, as shown by the graph in figure 2C.

In another embodiment, with reference to Figure 3A, B, C, the light diffusing tube 21 is 3 m long, has a diameter of 400 mm and is divided into 9 sections, I-IX. Going from the top end to the bottom end of said light diffusing tube, there is a first section I without slits 22, a second section with 20 slits 22, a third with 26, a fourth with 21, a fifth and a sixth section with about 15 slits, a seventh with 25, an octave and a ninth with 28.

Again, with reference to figure 3A, the length of the shorter side of said slits is about 5 mm in the second and third section, to pass to about 8 mm in the fourth, to about 16 mm in the fifth, to about 20 mm in the sixth, seventh and about 28 mm in the eighth and ninth sections. With said distribution of the slits, a light distribution of the type represented in figure 3B, 3C is obtained, where figure 3C represents a perspective view of a light diffusing tube which has a diameter of 400 mm, a length of 3 m, a development of 1212 mm. On the light diffusing tube 21 there are slits 22 and the emission spectrum 23 is indicated. The incoherent irradiation along the entire length of the tube remains almost constant, as shown by the graph in figure 3C.

In alternative embodiments, the light diffusing tubes according to the present invention are used in combination with each other, for example two 2 m light diffusing tubes are used, or a 2 m diffusing tube with a 3 m diffusing tube.

In an embodiment particularly advantageously, said ALS is placed at the top and / or at the bottom of a diffuser tube with the above characteristics.

In one embodiment, several light diffusing tubes are placed inside a PBR. By way of example, one or more of said light diffusing tubes are powered by artificial light, supplied by said ALS positioned at the top and / or at the bottom of said light diffusing tubes and one or more of said light diffusing tubes are powered with natural light.

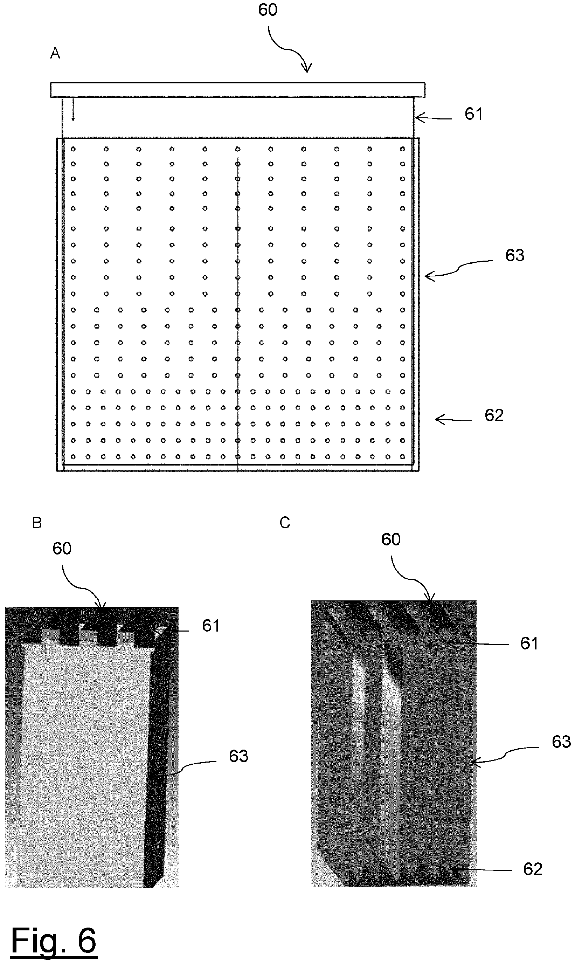

In one embodiment, said diffusing element is a light diffusing panel. With reference to Figure 6A, said metal film with slits is positioned on the walls of said light diffusing panel (60) in such a way that the interior of the light diffusing panel is defined by the metal film and the exterior of the light diffusing panel it is defined by the transparent material that constitutes the panel itself. Where said light diffusing panel is inserted in a PBR (63), there is a lower portion (62) of said panel, on the bottom of said PBR, and an upper portion (61), which emerges from said PBR. Conveniently, said slits are in a greater number on said lower portion. The upper portion, which emerges from said PBR, preferably has no slits, to avoid unwanted dispersion of the light outside the PBR.

Preferably, said slits are circular holes, preferably about 10 mm in diameter.

In one embodiment, said light diffusing panel has a thickness between 3 and 15 cm, a width between 70 and 120 cm, a height between 60 and 110 cm.

Figure 6B shows a perspective view of said light panel (60) immersed in said PBR (63), figure 6C a perspective view in section of the same.

A further object of the present invention is a method of controlling the lighting system. This method allows the control of the following parameters:
- the "PAR target", that is the minimum flux of photons necessary for the crop.
- the photoperiod of the cultures, that is the time of exposure of the algal culture to light.
- the individual light intensities of the three types of LEDs: RED, WHITE, and BLUE.
- the combination of light intensity between the different LEDs.

The indicated parameters make it possible to reproduce the light environment suitable for algal growth, in such a way that not only optimal growth is maintained but the production of useful molecules, such as antioxidants, is also increased.

It forms a further object of the present invention a lighting system which comprises:
- at least one ALS comprising a plurality of LEDs, independently controlled and providing three colours, RED, WHITE and BLUE;
- at least one light diffusing element closed at the top and / or at the bottom by said ALS;
- at least one PBR in which said at least one light diffusing element is immersed;
- light sensors positioned inside the PBR;
- an electronic device connected to said at least one ALS and to said light sensors;
- a computer.
where said system is characterized by being configured to combine the three colours together to obtain the desired spectrum, and where the intensity of each colour is independently modulated or controlled.

In a preferred form, said electronic device comprises:
- a microprocessor, which interacts with the computer;
- a memory to store the data used for setting up the system
- an output circuit and
- a power supply.

A further object of the present invention is a PBR which comprises the light system according to the present invention.

A further object of the present invention is a method for controlling the light system according to the present invention, characterized by the following steps:
a) Initialize the system parameters;
b) Define the PAR target;
c) Define the individual light intensities of the three types of LEDs: RED, WHITE and BLUE;
d) Define the combination of light intensity between the different LEDs;
e) Define the photoperiod of the cultures, i.e. the exposure time of the algal culture to light.
f) Read, through said light sensors, the amount of photons present in the photobioreactor, determining intensity and wavelength;
g) sending said signal f) to the microprocessor;
h) Compare said measure f) with the parameters defined in b), c), d) and e) and, if they differ,
i) Send on and / or off signal to the individual LEDs.

A further object of the present invention is a computer program which comprises a source code suitable for implementing the method described above.

A further object of the present invention is a computer program which contains the computer program as defined above.

Advantageously, in the case of suitable external light, the LEDs remain off. Otherwise, the software acts on the LEDs to compensate to reach the PAR target with a well-defined light spectrum.

The main advantage is to control the photoperiod even beyond the natural one to maximize production.

For example, in the case of spirulina it is possible to modulate the red, blue, and white LEDs, both in the ratio of the bands and in intensity, to have a greater growth of the alga in less time and then increase the blue component to 470 nm to stimulate the growth of phycocyanin, a precious blue pigment for food to be extracted from algal biomass.

The following examples have the sole purpose of better illustrating the invention, they are not intended to be in any way limitative of the same, the scope of which is defined by the claims.

### Experimental section

### Example 1: simulation parameters with OSRAM horticolture software

An ALS has been created according to the present invention comprising the LEDs indicated in table 1.

**Table 1**

| **Configuration and performance** | | | | | | |
|---|---|---|---|---|---|---|
| **Colour** | **product** | **Photon flux ratio** | **N° LED** | **Photon flux** | **Photosynthetic photonic flux** | **Radiant Flow** |
| Hyper Red (635-666 nm) | GH CSSRM3.24 | 32,8% | 90 pcs | 419 µmol/s | 417 µmol/s | 76 W |
| DeepBlue (425-455 nm) | C034UVH430 | 14,15 % | 40 pcs | 181 µmol/s | 180 µmol/s | 48 W |
| Blue (450-480 nm) | XPE2 L1-B50-M2 | 27,32 % | 40 pcs | 349 µmol/s | 348 µmol/s | 90 W |
| White | GW CSSRM2.P 3000K | 25,74 % | 80 pcs | 329 µmol/s | 319 µmol/s | 69 W |
| **Total** | | 100,01 % | 250 pcs | 1278 µmol/s | 1265 µmol/s | 283 W |

Said ALS, comprising a total of 250 LEDs, for a total photonic flux equal to 1278 µmol / s and a total photosynthetic photonic flux equal to 1265 µmol / s, was positioned at the top of a light diffusing tube with a diameter of 400 mm and a length of 2 m. Said light diffusing tube is divided into 8 sections, I-VIII. Going from the top end to the bottom end of said light diffusing tube, there is a first section without slits, a second section with 30 slits, a third with 36, a fourth with 25, a fifth with 26, a sixth section with 25 slits, a seventh with 32, an octave with 30. The length of the shorter side of said slits is about 5 mm in the second and third section, about 10 mm in the fourth, about 14 mm in the fifth, about 20 mm in the remaining three sections. Said diffuser tube was positioned in a PBR and the parameters reported in table 2 were measured. In particular, the radiant power of the system is equal to 283 W, the photon flux emission efficacy 2.72 µmol / J, with a 469 W electrical power and 60.3% conversion efficiency (Radiant Flux / Power Consumption).

**Table 2**

| **System power** | |
|---|---|
| Photonic flux (360-780nm) | 1278 µmol/s |
| Photonic flux efficacy (360-780nm) | 2,72 µmol/J |
| Photosynthetic photonic flux (400-700nm) | 1265 µmol/s |
| Photosynthetic photonic flux efficacy (400-700nm) | 2,7 µmol /J |
| Energy consumption | 469 W |

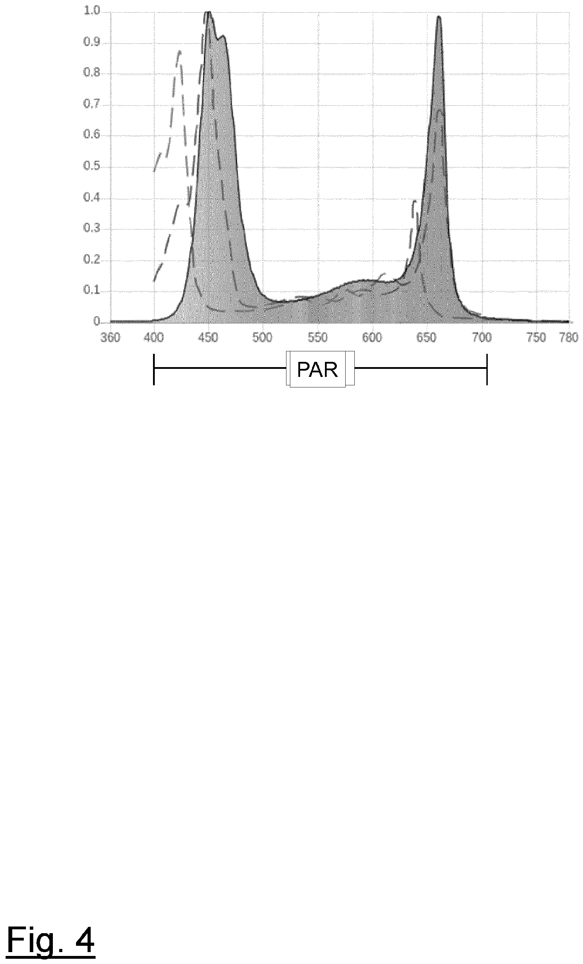

The resulting light spectrum is schematized in figure 4 where the active photosynthetic radiation (PAR) is highlighted.

## Claims

1. A kit for the lighting of a photobioreactor (PBR) which includes:
- at least one Artificial Lighting System (ALS) (1);
- at least one light diffusing element;
where said at least one ALS (1) comprises an external face (3) and an internal face (4), where on said external face (3) a plurality of blades (2) are placed and on said internal face (4) a plurality of LED modules (5) are placed, wherein said plurality comprises at least one LED in each of the RED, WHITE and BLUE channels;
where said at least one ALS (1) is positioned at the top and / or at the bottom of at least one of said light diffusing elements and said external face (3) faces outwards, said internal face (4) faces towards the interior of said light diffusing element and said ALS acts as a sealing element for said light diffusing element.

2. The kit according to claim 1, wherein said blades (2) are surface corrugated.

3. The kit according to claim 1 or 2, wherein said at least one light diffusing element is made of a material transparent to light and at least partially coated with a reflector which is a metal film with low thickness and high surface reflectance in which slits are cut.

4. The kit according to one of claims 1 to 3, wherein said diffusing element is a light diffusing tube.

5. The kit according to one of claims 1 to 4, wherein said diffusing element is a light diffusing panel.

6. The kit according to claim 4, wherein said light diffusing tube has a diameter greater than 300 mm, preferably between 350 and 800 mm, preferably about 400 mm and a length between 1.5 and 5 meters, preferably is about 2 meters long, or about 3 meters.

7. The kit according to claim 4, where said light diffusing tube is divided into at least 7 sections along the length of the tube itself, where said slits have homogeneous shape and size in each of said sections.

8. The kit according to claim 6 or 7, where said slits have a rectangular shape and the dimensions of the smaller side of said rectangle increase passing from the top sections of the light diffusing tube to the bottom sections, preferably the length of said minor side is between 3 and 30 mm, even more preferably between 5 and 20 mm.

9. The kit according to claim 5, where said light diffusing panel has a lower portion which, when inserted in said PBR, is located on the bottom of the same and an upper portion that emerges from said PCR, where said slits are in a greater number on said lower portion, preferably said upper portion has no slits.

10. The kit according to claim 9, wherein said slits are circular holes, preferably about 10 mm in diameter.

11. The kit according to claim 5, where said light diffusing panel has a thickness between 3 and 15 cm, a width between 70 and 120 cm, a height between 60 and 110 cm.

12. A lighting system that includes:
- at least one ALS comprising a plurality of LEDs, independently controlled and providing three colours, RED, WHITE and BLUE;
- at least one light diffusing element closed at the top and / or at the bottom by said ALS;
- at least one PBR in which said at least one light diffusing element is immersed;
- light sensors positioned inside the PBR;
- an electronic device connected to said at least one ALS and to said light sensors;
- a computer;
where said system is **characterized by** being configured to combine the three colours together to obtain the desired spectrum, and where the intensity of each colour is independently modulated or controlled.

13. A method for controlling the lighting system according to claim 12, **characterized by** the following steps:
a) Initialize the system parameters;
b) Define the PAR target;
c) Define the individual light intensities of the three types of LEDs: RED, WHITE and BLUE;
d) Define the combination of light intensity between the different LEDs;
e) Define the photoperiod of the cultures, i.e. the exposure time of the algal culture to light;
f) Read, through said light sensors, the amount of photons present in the photobioreactor, determining intensity and wavelength;
g) sending said signal f) to the microprocessor;
h) Compare said measure f) with the parameters defined in b), c), d) and e) and, if they differ,
i) Send on and / or off signal to the individual LEDs.
